# EUROPEAN PATENT SPECIFICATION

(11) **EP 1 446 099 B1**
(45) Date of publication and mention of the grant of the patent: **15.07.2009**
(21) Application number: 02784472.9
(22) Date of filing: 14.11.2002
(51) Int. Cl.: A61K 9/00, A61K 47/34

(54) **INJECTABLE DEPOT COMPOSITION**
INJIZIERBARE DEPOTZUSAMMENSETZUNGEN
COMPOSITION POUR INJECTION RETARD

(30) Priority: 14.11.2001 US 336254 P
(43) Date of publication of application: 18.08.2004
(73) Proprietor: Durect Corporation, Cupertino, CA 95014 (US)
(72) Inventor: CHEN, Guohua, Sunnyvale, CA 94086 (US); HOUSTON, Paul, Ricky, Hayward, CA 94542 (US); WRIGHT, Jeremy, Corwin, Los Altos, CA 94024-4135 (US); FRANSSON, Jonas, S-752 38 Uppsala (SE); HGERTMAN, Birger, S-165 77 Hasselby (SE)
(74) Representative: Bradley, Adrian
(86) International application number: PCT/US2002/036717
(87) International publication number: WO 2003/041685

(56) References cited:
- WO-A-00/74650
- WO-A-99/47073
- US-A- 4 938 763
- US-A- 5 447 725
- US-A- 5 674 292
- US-A- 6 130 200
- US-B1- 6 193 991
- US-B1- 6 423 818
- US-B1- 6 451 346

## Description

### BACKGROUND OF THE INVENTION

### Field of the Invention

The present invention relates generally to gel compositions that can be utilized as carriers for beneficial agents when injected into a subject and which can provide sustained release of the beneficial agent over time. More particularly, it relates to gel compositions such as described above that contain an agent that renders the composition thixotropic to facilitate injection of the gel into a subject with minimal discomfort to the subject.

### Description of the Related Art

Biodegradable polymers have been used for many years in medical applications. The majority of these biodegradable polymers have been based upon glycolide, lactide, caprolactone, and copolymers thereof. Within the last decade, greater emphasis has been placed on the use of injectable polymer compositions that provide a depot of beneficial agent for dispensing to a subject over time.

One way to avoid the incision needed to implant drug delivery systems is to inject them as small particles, microspheres, or microcapsules. For example, U.S. Patent No. 5,019,400 describes the preparation of controlled release microspheres via a very low temperature casting process. These materials may or may not contain a drug which can be released into the body. Although these materials can be injected into the body with a syringe, they do not always satisfy the demand for a biodegradable implant.

Because they are particulate in nature, they do not form a continuous film or solid implant with the structural integrity needed for certain prostheses. When inserted into certain body cavities such as a mouth, a periodontal pocket, the eye, or the vagina where there is considerable fluid flow, these small particles, microspheres, or microcapsules are poorly retained because of their small size and discontinuous nature. Further, the particles tend to aggregate and thus their behavior is hard to predict. In addition, microspheres or microcapsules prepared from these polymers and containing drugs for release into the body are sometimes difficult to produce on a large scale, and their storage and injection characteristics present problems. Furthermore, one other major limitation of the microcapsule or small-particle system is their lack of reversibility without extensive surgical intervention. That is, if there are complications after they have been injected, it is considerably more difficult to remove them from the body than with solid implants. A still further limitation on microparticles or microcapsulation is the difficulty in encapsulating protein and DNA-based drugs without degradation caused by solvents and temperature extremes.

The art has developed various drug delivery systems in response to the aforementioned challenges. For instance, U.S. Patent No. 4,938,763 and its divisional U.S. Patent No. 5,278,201 relate to a biodegradable polymer for use in providing syringeable, in-situ forming, solid biodegradable implants for animals. In one embodiment, a thermoplastic system is used wherein a nonreactive polymer is dissolved in a biocompatible solvent to form a liquid which is placed in the animal wherein the solvent dissipates to produce the solid implant. Alternatively, a thermosetting system is used wherein effective amounts of a liquid acrylic ester-terminated, biodegradable prepolymer and a curing agent are formed and the liquid mixture is placed within the animal wherein the prepolymer cures to form the solid implant. It is stated that the systems provide a syringeable, solid biodegradable delivery system by the addition of an effective level of a biologically active agent to the liquid before the injection into the animal.

### BRIEF SUMMARY OF THE INVENTION

Gel compositions are provided that can be utilized as carriers for beneficial agents when injected into a subject and which can provide sustained release of the beneficial agent over time. In particular, gel compositions containing an agent that renders the composition thixotropic to facilitate injection of the gel into a subject with minimal discomfort to the subject are described.

U.S. Patent No. 5,242,910 describes a sustained release composition for treating periodontal disease. The composition comprises copolymers of lactide and glycolide, triacetin (as a solvent/plasticizer) and an agent providing relief of oral cavity diseases. The composition can take the form of a gel and can be inserted into a periodontal cavity via a syringe using either a needle or a catheter. As additional optional components, the composition can contain surfactants, flavoring agents, viscosity controlling agents, complexing agents, antioxidants, other polymers, gums, waxes/oils, and coloring agents. One illustrative viscosity controlling agent set forth in one of the examples is polyethylene glycol 400. U.S. Patent Nos. 5,620,700 and 5,556,905 relate to polymer compositions for injectable implants using solvents and/or plasticizers.

Prior art polymer compositions for injectable implants have used solvent/plasticizers that are very or relatively soluble in aqueous body fluids to promote rapid solidification of the polymer at the implant site and promote diffusion of drug from the implant. However, it has now been observed that a serious problem associated with prior art polymeric implants utilizing water soluble polymer solvents is the rapid migration of water into the polymer composition when the implant is placed in the body and exposed to aqueous body fluids. That characteristic often results in uncontrolled release of beneficial agent that is manifested by an initial, rapid release of beneficial agent from the polymer composition, corresponding to a "burst" of beneficial agent being released from the implant. The burst often results in a substantial portion of the beneficial agent, if not all, being released in a very short time, e.g., hours or 1-2 days. Such an effect can be unacceptable, particularly in those circumstances where sustained delivery is desired, i.e., delivery of beneficial agent over a period of a week or a month or more, or where there is a narrow therapeutic window and release of excess beneficial agent can result in adverse consequences to the subject being treated, or where it is necessary to mimic the naturally-occurring daily profile of beneficial agents, such as hormones and the like, in the body of the subject being treated.

In an attempt to control burst and modulate and stabilize the delivery of the beneficial agent the prior art has coated particles of beneficial agent to retard release into an aqueous environment and extend release of the beneficial agent over time. Alternatively, various stabilizing or release modulating agents, such as metal salts as described in U.S. Patents 5,656,297, 5,654,010, 4,985,404 and 4,853,218 have been used. U.S. Patent No. 3,923,939 describes a method of reducing initial burst of an active agent from a delivery device by removing, prior to implantation, active agent from the exterior surface of the delivery device and through a layer of at least 5% of the overall body thickness extending from the exterior surface of the device.

Notwithstanding some success, those methods have not been entirely satisfactory for the large number of beneficial agents that would be effectively delivered by implants, since in many instances the modulation and stabilization effect is the result of the formation of a complex of the metal ion with the beneficial agent. When such complexes do not form, the stabilization/modulation effect may not be adequate to prevent undesirable "burst" of the beneficial agent upon its introduction into the implant site.

U.S. Patent No. 6,130,200 describes a viscous gel carrier for a beneficial agent that can be injected into a subject. Use of a viscous gel lowers the initial burst of beneficial agent that is often seen with injectable depot systems. Notwithstanding the many advantages of the system described in the patent, in certain applications the viscosity of the gel may be so high as to result in relatively high injection forces required to dispense the gel from a syringe.

International application WO 98/27962 describes the formation of a thixotropic gel composition that provides for shear thinning and more acceptable injectability of the gel, such that lower injection forces are needed to expel the gel from a syringe and also lower the likelihood of substantial discomfort to a subject by use of smaller needles than would otherwise be required. While the systems described therein provide suitable depot systems for many applications, the described system utilized relatively large amounts of emulsifying agent, e.g., about one-third of the total weight of the composition. It has been discovered that in certain systems smaller quantities of certain compounds may be mixed with lactic acid-based polymers and a suitable solvent for the polymer that modify the flow characteristics of the gel formed, without the formation of an emulsion but still resulting in thixotropic compositions that are readily injectable through needles having a gauge that when used is not unduly uncomfortable to a subject. Also, use of such smaller amounts of an agent that imparts thixotropic properties to the gel may allow for smaller depot volume and mass without diminishing delivery of a required amount of beneficial agent over a prolonged period of time for an intended therapeutic effect.

### SUMMARY OF THE INVENTION

In one aspect the invention comprises a composition comprising a lactic acid-based polymer, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent. The lower alkanols are straight or branched chain alcohols having 2-6 carbon atoms as exemplified by ethanol, propanol, isopropanol and the like. A preferred agent is ethanol. The composition may include an amount of ethanol that is greater than or equal to 0.01 weight percent and less than or equal to 15 weight percent of the combined weight of the solvent and the agent. The composition may include an amount of ethanol that is greater than or equal to 0.1 weight percent and less than or equal to 5 weight percent of the combined weight of the solvent and the agent. The composition may include an amount of ethanol that is greater than or equal to 0.5 weight percent and less than or equal to 5 weight percent of the combined weight of the solvent and the agent.

In another aspect, the invention comprises a composition comprising polylactic acid polymer, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent. The lower alkanols are straight or branched chain alcohols having 2-6 carbon atoms as exemplified by ethanol, propanol, isopropanol and the like. A preferred agent is ethanol. The composition may include an amount of ethanol that is greater than or equal to 0.01 weight percent and less than or equal to 15 weight percent of the combined weight of the solvent and the agent. The composition may include an amount of ethanol that is greater than or equal to 0.1 weight percent and less than or equal to 5 weight percent of the combined weight of the solvent and the agent. The composition may include an amount of ethanol that is greater than or equal to 0.5 weight percent and less than or equal to 5 weight percent of the combined weight of the solvent and the agent. The polylactic acid polymer may have a weight average molecular weight in the range of about 1,000 to about 120,000; preferably about 5,000 to about 50,000; and more preferably about 8,000 to about 30,000.

In yet another aspect, the invention comprises a composition comprising a lactic acid-based polymer formed as a copolymer of lactic acid and glycolic acid, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent. The lower alkanols are straight or branched chain alcohols having 2-6 carbon atoms as exemplified by ethanol, propanol, isopropanol and the like. A preferred agent is ethanol. The composition may include an amount of ethanol that is greater than or equal to 0.01 weight percent and less than or equal to 15 weight percent of the combined weight of the solvent and the agent. The composition may include an amount of ethanol that is greater than or equal to 0.1 weight percent and less than or equal to 5 weight percent of the combined weight of the solvent and the agent. The composition may include an amount of ethanol that is greater than or equal to 0.5 weight percent and less than or equal to 5 weight percent of the combined weight of the solvent and the agent. The weight average molecular weight of the copolymer may be in the range of 1,000 to about 120,000; preferably about 5,000 to about 50,000; and more preferably about 8,000 to about 30,000.

In another aspect, the invention comprises a method of administering, locally or systemically, a beneficial agent to a subject which comprises implanting beneath the subject's body surface a composition as described above. Preferably, the system releases 40% or less by weight of the beneficial agent present in the viscous gel within the first 24 hours after implantation in the subject. More preferably, 30% or less by weight of the beneficial agent will be released within the first 24 hours after implantation, and the implanted composition has a burst index of 12 or less, preferably 8 or less.

In another aspect, the invention pertains to a composition and a method of administering such composition as described above, wherein the beneficial agent is selected from a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents, antiproliferative agents, antimitotic agents, angiogenic agents, anticoagulants, fibrinolytic agents, growth factors, antibodies, ocular drugs, and metabolites, analogs, derivatives, fragments, and purified, isolated, recombinant and chemically synthesized versions of these species. In preferred embodiments, the beneficial agent is human growth hormone, methionine-human growth hormone; des-phenylalanine human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor, filgrastim epidermal growth factors (EGFs), platelet-derived growth factor (PDGFs), insulin-like growth factors (IGFs), fibroblast-growth factors (FGFs), transforming-growth factors (TGFs), interleukins (ILs), colony-stimulating factors (CSFs, MCFs, GCSFs, GMCSFs), Interferons (IFNs), endothelial growth factors (VEGF, EGFs), erythropoietins (EPOs), angiopoietins (ANGs), placenta-derived growth factors (PIGFs), and hypoxia induced transcriptional regulators (HIFs). Preferably, the beneficial agent is present in an amount of from 0.1 to 50% by weight of the combined amounts of the polymer, the solvent and the beneficial agent. In preferred embodiments, the beneficial agent is in the form of particles dispersed or dissolved in the viscous gel, wherein the beneficial agent is in the form of particles having an average particle size of from 0.1 to 250 microns. In certain preferred embodiments, the beneficial agent is in the form of particles wherein the particle further comprises a component selected from the group consisting of a stabilizing agent, bulking agent, chelating agent and a buffering agent.

### BRIEF DESCRIPTION OF THE DRAWINGS

The foregoing and other objects, features and advantages of the present invention will be more readily understood upon reading the following detailed description in conjunction with the drawings in which:

Figure 1 is a graph illustrating the relationship of viscosity and shear rate for various compositions of the present invention as compared to a prior art composition (formulations 1-4);

Figure 2 is a graph illustrating the relationship of viscosity and shear rate for various compositions of the present invention as compared to a prior art composition (formulations 5-8);

Figure 3 is a graph illustrating the relationship between force required to inject compositions of the present invention (formulations 1-4) from a syringe using a 20 gauge needle and the percentage of ethanol in the polymer solvent;

Figures 4A and 4B are graphs illustrating the relationship between force required to inject compositions of the present invention (formulations 9-16) from a syringe using a 24 gauge needle and the percentage of ethanol in the polymer solvent;

Figure 5 is a graph illustrating the in vivo release profile of human growth hormone obtained from various depot formulations, including those of the present invention (formulations 5-8);

Figures 6A and 6B are graphs illustrating the in vivo release profile of human growth hormone obtained from various depot formulations, including those of the present invention (formulations 9-16);

Figure 7 shows the burst index profile profiling the release of human growth hormone ("hGH") from formulations 5-8 observed in rats; and

Figure 8 shows the burst index profile profiling the release of human growth hormone ("hGH") from formulations 6 and 7 and the Neutropin^{®} depot formulation observed in rats.

### DETAILED DESCRIPTION OF THE INVENTION

### Overview and Definitions:

The present invention is directed to gel compositions that can be utilized as carriers for beneficial agents when injected into a subject and which can provide sustained release of the beneficial agent over time. In particular, the invention is directed to gel compositions such as described above that contain an agent that renders the composition thixotropic to facilitate injection of the gel into a subject with minimal discomfort to the subject.

In describing and claiming the present invention, the following terminology will be used in accordance with the definitions set out below.

The singular forms "a," "an" and "the" include plural referents unless the context clearly dictates otherwise. Thus, for example, reference to "a solvent" includes a single solvent as well as a mixture of two or more different solvents, reference to "a beneficial agent" includes a single beneficial agent as well as two or more different beneficial agents in combination, reference to "an alcohol" includes a single alcohol as well as a mixture of two or more different alcohols, and the like.

The term "beneficial agent" means an agent that effects a desired beneficial, often pharmacological, effect upon administration to a human or an animal, whether alone or in combination with other pharmaceutical excipients or inert ingredients.

As used herein, the term "polynucleotide" refers to a polymeric form of nucleotides of any length, either ribonucleotides or deoxyribonucleotides, and includes double- and single-stranded DNA and RNA. It also includes known types of modifications, substitutions, and internucleotide modifications, which are known in the art.

As used herein, the term "recombinant polynucleotide" refers to a polynucleotide of genomic, cDNA, semisynthetic, or synthetic origin which, by virtue of its origin or manipulation: is not associated with all or a portion of a polynucleotide with which it is associated in nature; is linked to a polynucleotide other than that to which it is linked in nature; or does not occur in nature.

As used herein, the term "polypeptide" refers to a polymer of amino acids, inlcuding for example, peptides, oligopeptides, and proteins and derivatives, analogs and fragments thereof, as well as other modifications known in the art, both naturally occurring and non-naturally occurring.

As used herein, the term "purified" and "isolated" when referring to a polypeptide or nucleotide sequence means that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type present.

The term "AUC" means the area under the curve obtained from an in vivo assay in a subject by plotting blood plasma concentration of the beneficial agent in the subject against time, as measured from the time of implantation of the composition, to a time "t" after implantation. The time t will correspond to the delivery period of beneficial agent to a subject.

The term "burst index" means, with respect to a particular composition intended for systemic delivery of a beneficial agent, the quotient formed by dividing (i) the AUC calculated for the first time period after implantation of the composition into a subject divided by the number of hours in the first time period (t₁), by (ii) the AUC calculated for the time period of delivery of beneficial agent, divided by the number of hours in the total duration of the delivery period (t₂). For example the burst index at 24 hours is the quotient formed by dividing (i) the AUC calculated for the first twenty-four hours after implantation of the composition into a subject divided by the number 24, by (ii) the AUC calculated for the time period of delivery of beneficial agent, divided by the number of hours in the total duration of the delivery period.

The phrase "dissolved or dispersed" is intended to encompass all means of establishing a presence of beneficial agent in the gel composition and includes dissolution, dispersion, suspension and the like.

The term "systemic" means, with respect to delivery or administration of a beneficial agent to a subject, that the beneficial agent is detectable at a biologically-significant level in the blood plasma of the subject.

The term "local" means, with respect to delivery or administration of a beneficial agent to a subject, that the beneficial agent is delivered to a localized site in the subject but is not detectable at a biologically significant level in the blood plasma of the subject.

The term "gel vehicle" means the composition formed by mixture of the polymer and solvent in the absence of the beneficial agent.

The term "prolonged period" means a period of time over which release of a beneficial agent from the implant of the invention occurs, which will generally be about one week or longer, and preferably about 30 days or longer.

The term "initial burst" means, with respect to a particular composition of this invention, the quotient obtained by dividing (i) the amount by weight of beneficial agent released from the composition in a predetermined initial period of time after implantation, by (ii) the total amount of beneficial agent that is to be delivered from an implanted composition. It is understood that the initial burst may vary depending on the shape and surface area of the implant. Accordingly, the percentages and burst indices associated with initial burst described herein are intended to apply to compositions tested in a form resulting from dispensing of the composition from a standard syringe.

The term "solubility modulator" means, with respect to the beneficial agent, an agent that will alter the solubility of the beneficial agent, with reference to polymer solvent or water, from the solubility of beneficial agent in the absence of the modulator. The modulator may enhance or retard the solubility of the beneficial agent in the solvent or water. However, in the case of beneficial agents that are highly water soluble, the solubility modulator will generally be an agent that will retard the solubility of the beneficial agent in water. The effects of solubility modulators of the beneficial agent may result from interaction of the solubility modulator with the solvent, or with the beneficial agent itself, such as by the formation of complexes, or with both. For the purposes hereof, when the solubility modulator is "associated" with the beneficial agent, all such interactions or formations as may occur are intended. Solubility modulators may be mixed with the beneficial agent prior to its combination with the viscous gel or may be added to the viscous gel prior to the addition of the beneficial agent, as appropriate.

The terms "subject" and "patient" mean, with respect to the administration of a composition of the invention, an animal or a human being.

The term "bioerodible" refers to a material that gradually decomposes, dissolves, hydrolyzes and/or erodes in situ. Generally, the "bioerodible" polymers herein are polymers that are hydrolyzable, and bioerode in situ primarily through hydrolysis.

The term "thixotropic" is used in its conventional sense to refer to a gel composition that can liquefy or at least exhibit a decrease in apparent viscosity upon application of mechanical force such as shear force. A "thixotropic agent" as used herein is one that increases the thixotropy of the composition in which it is contained, promoting shear thinning and enabling use of reduced injection force.

The polymer, solvent and other agents of the invention must be "biocompatible"; that is they must not cause irritation, inflammation or necrosis in the environment of use. The environment of use is a fluid environment and may comprise a subcutaneous or intramuscular portion or body cavity of a human or animal.

### Injectable Depot Formulations:

As described previously, injectable depot formulations for delivery of beneficial agents over a prolonged period of time may be formed as viscous gels prior to injection of the depot into a subject. The viscous gel supports dispersed beneficial agent to provide appropriate delivery profiles, which include those having low initial burst, of the beneficial agent as the beneficial agent is released from the depot over time.

Typically, the viscous gel will be injected from a standard hypodermic syringe that has been pre-filled with the beneficial agent-viscous gel composition as the depot. It is often preferred that injections take place using the smallest size needle (i.e., smallest diameter) to reduce discomfort to the subject when the injection takes place through the skin and into subcutaneous tissue. It is desirable to be able to inject gels through needles ranging from 16 gauge and higher, preferably 20 gauge and higher, more preferably 22 gauge and higher, even more preferably 24 gauge and higher. With highly viscous gels, i.e., gels having a viscosity of about 200 poise or greater, injection forces to dispense the gel from a syringe having a needle in the 20-30 gauge range may be so high as to make the injection difficult or reasonably impossible when done manually. At the same time, the high viscosity of the gel is desirable to maintain the integrity of the depot after injection and during the dispensing period and also facilitate desired suspension characteristics of the beneficial agent in the gel.

A thixotropic gel exhibits reduced viscosity when subjected to shear force. The extent of the reduction is in part a function of the shear rate of the gel when subjected to the shearing force. When the shearing force is removed, the viscosity of the thixotropic gel returns to a viscosity at or near that which it displayed prior to being subjected to the shearing force. Accordingly, a thixotropic gel may be subjected to a shearing force when injected from a syringe which temporarily reduces its viscosity during the injection process. When the injection process is completed, the shearing force is removed and the gel returns very near to its previous state.

A composition of a polymer and polymer solvent that includes an agent that imparts thixotropic characteristics to the viscous gel formed by the polymer solvent and polymer provides the desired advantages noted above. It is additionally desirable to use the agent in amounts that are sufficiently small so as not to unnecessarily increase the mass and volume of the depot that is to be injected. In this regard it is desirable that the agent, i.e. lower alkanols, particularly ethanol, is not a polymer solvent. As is described more fully below, the addition of small amounts of lower alkanols, especially ethanol, to polymer depots formed as viscous gels from lactic acid-based polymers and suitable polymer solvents provide the foregoing desirable characteristics in compositions of the invention described here.

The polymer may be a polylactide, that is, a lactic acid-based polymer that can be based solely on lactic acid or can be a copolymer based on lactic acid and glycolic acid which may include small amounts of other comonomers that do not substantially affect the advantageous results which can be achieved in accordance with the present invention. As used herein, the term "lactic acid" includes the isomers L-lactic acid, D-lactic acid, DL-lactic acid and lactide while the term "glycolic acid" includes glycolide. The polymer may have a monomer ratio of lactic acid/glycolic acid of from about 100:0 to about 15:85, preferably from about 75:25 to about 30:70, more preferably from about 60:40 to about 40:60, and an especially useful copolymer has a monomer ratio of lactic acid/glycolic acid of about 50:50.

The lactic acid-based polymer has a number average molecular weight of from about 1,000 to about 120,000, preferably from about 5,000 to about 50,000, more preferably from about 8,000 to about 30,000, as determined by gel permeation chromatography (GPC). As indicated in aforementioned U.S. Patent No. 5,242,910, the polymer can be prepared in accordance with the teachings of U.S. Patent No. 4,443,340. Alternatively, the lactic acid-based polymer can be prepared directly from lactic acid or a mixture of lactic acid and glycolic acid (with or without a further comonomer) in accordance with the techniques set forth in U.S. Patent No. 5,310,865. The contents of all of these patents are incorporated by reference. Suitable lactic acid-based polymers are available commercially. For instance, 50:50 lactic acid:glycolic acid copolymers having molecular weights of 8,000, 10,000, 30,000 and 100,000 are available from Boehringer Ingelheim (Petersburg, VA), Medisorb Technologies International L.P. (Cincinatti, OH) and Birmingham Polymers, Inc. (Birmingham, AL) as described below.

Examples of polymers include, but are not limited to, Poly (D,L-lactide) Resomer^{®} L104, PLA-L104, Poly (D,L-lactide-co-glycolide) 50:50 Resomer^{®} RG502, Poly (D,L-lactide-co-glycolide) 50:50 Resomer^{®} RG502H, PLGA-502H, Poly (D,L-lactide-co-glycolide) 50:50 Resomer^{®} RG503, PLGA-503, Poly (D,L-lactide-co-glycolide) 50:50 Resomer^{®} RG506, PLGA-506, Poly (D,L-lactide-co-glycolide) 50:50 Resomer^{®} RG755, PLGA-755, Poly L-Lactide MW 2,000 (Resomer^{®} L 206, Resomer^{®} L 207, Resomer^{®} L 209, Resomer^{®} L 214); Poly D,L Lactide (Resomer^{®} R 104, Resomer^{®} R 202, Resomer^{®} R 203, Resomer^{®} R 206, Resomer^{®} R 207, Resomer^{®} R 208); Poly L-Lactide-co-D,L-lactide 90:10 (Resomer^{®} LR 209); Poly glycolide (Resomer^{®} G 205); Poly D,L-lactide-co-glycolide 50:50 (Resomer^{®} RG 504 H, Resomer^{®} RG 504, Resomer^{®} RG 505); Poly D-L-lactide-co-glycolide 75:25 (Resomer^{®} RG 752, Resomer^{®} RG 756); Poly D,L-lactide-co-glycolide 85:15 (Resomer^{®} RG 858); Poly L-lactide-co-trimethylene carbonate 70:30 (Resomer^{®} LT 706); Poly dioxanone (Resomer^{®} X 210) (Boehringer Ingelheim Chemicals, Inc., Petersburg, VA).

Additional examples include, but are not limited to, DL-lactide/glycolide 100:0 (MEDISORB^{®} Polymer 100 DL High, MEDISORB^{®} Polymer 100 DL Low); DL-lactide/ glycolide 85/15 (MEDISORB^{®} Polymer 8515 DL High, MEDISORB^{®} Polymer 8515 DL Low); DL-lactide/glycolide 75/25 (MEDISORB^{®} Polymer 7525 DL High, MEDISORB^{®} Polymer 7525 DL Low); DL-lactide/glycolide 65/35 (MEDISORB^{®} Polymer 6535 DL High, MEDISORB^{®} Polymer 6535 DL Low); DL-lactide/glycolide 54/46 (MEDISORB^{®} Polymer 5050 DL High, MEDISORB^{®} Polymer 5050 DL Low); and DL-lactide/glycolide 54/46 (MEDISORB^{®} Polymer 5050 DL 2A(3), MEDISORB^{®} Polymer 5050 DL 3A(3), MEDISORB^{®} Polymer 5050 DL 4A(3)) (Medisorb Technologies International L.P., Cincinatti, OH); and Poly D,L-lactide-co-glycolide 50:50; Poly D,L-lactide-co-glycolide 65:35; Poly D,L-lactide-co-glycolide 75:25; Poly D,L-lactide-co-glycolide 85:15; Poly DL-lactide; Poly L-lactide; Poly glycolide; Poly ε-caprolactone; Poly DL-lactide-co-caprolactone 25:75; and Poly DL-lactide-co-caprolactone 75:25 (Birmingham Polymers, Inc., Birmingham, AL).

The biocompatible 5 to about 90% by weight, preferably from about 10 to about 85% by weight, preferably from about 15 to about 80% by weight, preferably from about 20 to about 75% by weight, preferably from about 30 to about 70% by weight and typically from about 35 to about 65% by weight of the viscous gel, the viscous gel comprising the combined amounts of the biocompatible polymer and the solvent.

The solvent must be biocompatible and is selected so as to dissolve the polymer to form a viscous gel that can maintain particles of the beneficial agent dissolved or dispersed and isolated from the environment of use prior to release. The solvent is selected from the group consisting of lower alkyl and aralkyl esters of benzoic acid, such as benzyl benzoate, methyl benzoate, ethyl benzoate and the like.

The solvent typically is present in an amount of from about 95 to about 20% by weight and is preferably present in an amount of from about 80 to about 50% by weight and often 65 to 35% by weight of the viscous gel, that is the combined amounts of the polymer and the solvent. The viscous gel formed by mixing the polymer and the solvent typically exhibits a viscosity of from about 100 to about 200,000 poise, preferably from about 500 to about 50,000 poise often from about 1,000 to about 50,000 poise measured at a 1 sec⁻¹ shear rate and 25°C using a Haake Rheometer at about 1-2 days after mixing is completed. Mixing the polymer with the solvent can be achieved with conventional low shear equipment such as a Ross double planetary mixer for from about 1 to about 2 hours.

The agent that imparts thixotropic properties to the polymer gel is selected from the lower alkanols. Lower alkanol means an alcohol that contains 2-6 carbon atoms and is straight chain or branched chain. Such alcohols may be exemplified by ethanol, isopropanol, and the like. Importantly, such an agent that imparts thixotropic properties to the polymer gel is not a polymer solvent. (See e.g., Development of an in situ forming bidegradable poly-lactide-co-glycolide system for controlled release of proteins, Lambert, W.J., and Peck, K.D., Journal of Controlled Release, 33 (1995) 189-195).

Surprisingly, only a very small amount of agent need be added to the polymer solution of the polymer and polymer solvent to obtain the desired reduction in injection force when the gel is dispensed from a syringe. Accordingly, an amount of agent that is less than 15% by weight of the combined weight of the polymer solvent and the agent has been found to be satisfactory. The agent may be present in amounts of 0.01 to 15 weight percent, preferably in amounts of 0.1 to 5 weight percent, and often in amounts of 0.5 to 5 weight percent of the combined weight of the solvent and the agent.

The beneficial agent can be any physiologically or pharmacologically active substance or substances optionally in combination with pharmaceutically acceptable carriers and additional ingredients such as antioxidants, stabilizing agents, permeation enhancers, etc. that do not substantially adversely affect the advantageous results that can be attained by the present invention. The beneficial agent may be any of the agents which are known to be delivered to the body of a human or an animal and that are preferentially soluble in water rather than in the polymer-dissolving solvent. These agents include drug agents, medicaments, vitamins, nutrients, or the like. Included among the types of agents which meet this description are nutrients, vitamins, food supplements, sex sterilants, fertility inhibitors and fertility promoters.

Drug agents which may be delivered by the present invention include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents including anti-inflammatory corticosteroids, antiproliferative agents, antimitotic agents, angiogenic agents, anticoagulants, fibrinolytic agents, growth factors, antibodies, ocular drugs, and metabolites, analogs (including synthetic and substituted analogs), derivatives (including aggregative conjugates/fusion with other macromolecules and covalent conjugates with unrelated chemical moieties by means known in the art) fragments, and purified, isolated, recombinant and chemically synthesized versions of these species.

Examples of drugs that may be delivered by the composition of the present invention include, but are not limited to, procaine, procaine hydrochloride, tetracaine, tetracaine hydrochloride, cocaine, cocaine hydrochloride, chloroprocaine, chloroprocaine hydrochloride, proparacaine, proparacaine hydrochloride, piperocaine, piperocaine hydrochloride, hexylcaine, hexylcaine hydrochloride, naepaine, naepaine hydrochloride, benzoxinate, benzoxinate hydrochloride, cyclomethylcaine, cyclomethylcaine hydrochloride, cyclomethylcaine sulfate, lidocaine, lidocaine hydrochloride, bupivicaine, bupivicaine hydrochloride, mepivicaine, mepivacaine hydrochloride, prilocaine, prilocaine hydrochloride, dibucaine and dibucaine hydrochloride, etidocaine, benzocaine, propoxycaine, dyclonin, pramoxine, oxybuprocaine, prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17α-hydroxyprogesterone acetate, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat, captopril, ramipril, famotidine, nizatidine, sucralfate, etintidine, tetratolol, minoxidil, chlordiazepoxide, diazepam, amitriptyline, and imipramine. Further examples are proteins and peptides which include, but are not limited to, bone morphogenic proteins, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferons such as interferon alpha-2a, interferon alpha-2b, and consensus interferon, interleukins, growth factors such as epidermal growth factors (EGF), platelet-derived growth factors (PDGF), fibroblast growth factors (FGF), transforming growth factors-α (TGF-α), transforming growth factors-β (TGF-β), erythropoietin (EPO), insulin-like growth factor-I (IGF-I), insulin-like growth factor-II (IGF-II), interleukin-1, interleukin-2, interleukin-6, interleukin-8, tumor necrosis factor-α (TNF-α), tumor necrosis factor-β (TNF-β), Interferon-α (INF-α), Interferon-β (INF-β), Interferon-γ (INF-γ), Interferon-ω (INF-ω), colony stimulating factors (CGF), vascular cell growth factor (VEGF), thrombopoietin (TPO), stromal cell-derived factors (SDF), placenta growth factor (PIGF), hepatocyte growth factor (HGF), granulocyte macrophage colony stimulating factor (GM-CSF), glial-derived neurotropin factor (GDNF), granulocyte colony stimulating factor (G-CSF), ciliary neurotropic factor (CNTF), bone morphogeneic proteins (BMP), coagulation factors, human pancreas hormone releasing factor, analogs and derivatives of these compounds, and pharmaceutically acceptable salts of these compounds, or their analogs or derivatives.

In certain preferred embodiments, the beneficial agent includes chemotactic growth factors, proliferative growth factors, stimulatory growth factors, and transformational peptide growth factors including genes, precursors, post-translational-variants, metabolites, binding-proteins, receptors, receptor agonists and antagonists of the following growth factor families: epidermal growth factors (EGFs), platelet-derived growth factor (PDGFs), insulin-like growth factors (IGFs), fibroblast-growth factors (FGFs), transforming-growth factors (TGFs), interleukins (ILs), colony-stimulating factors (CSFs, MCFs, GCSFs, GMCSFs), Interferons (IFNs), endothelial growth factors (VEGF, EGFs), erythropoietins (EPOs), angiopoietins (ANGs), placenta-derived growth factors (PIGFs), and hypoxia induced transcriptional regulators (HIFs).

To the extent not mentioned in the previous paragraph, the beneficial agents described in aforementioned U.S. Patent No. 5,242,910 can also be used. One particular advantage of the present invention is that materials, such as proteins, as exemplified by the enzyme lysozyme, and cDNA, and DNA incorporated into vectors both viral and nonviral, which are difficult to microcapsulate or process into microspheres can be incorporated into the compositions of the present invention without the level of degradation experienced with other techniques.

The beneficial agent is preferably incorporated into the viscous gel formed from the polymer and the solvent in the form of particles typically having an average particle size of from about 0.1 to about 250 microns, preferably from about 1 to about 200 microns and often from 30 to 125 microns.

To form a suspension of particles of the beneficial agent in the viscous gel formed from the polymer and the solvent, any conventional low shear device can be used such as a Ross double planetary mixer at ambient conditions. In this manner, efficient distribution of the beneficial agent can be achieved substantially without degrading the beneficial agent.

The beneficial agent is typically dissolved or dispersed in the composition in an amount of from about 0.1 % to about 50% by weight, preferably in an amount of from about 1% to about 40%, more preferably in an amount of about 2% to about 30%, and often 2 to 20% by weight of the combined amounts of the polymer, solvent, agent and beneficial agent. Depending on the amount of beneficial agent present in the composition, one can obtain different release profiles. More specifically, for a given polymer and solvent, by adjusting the amounts of these components and the amount of the beneficial agent, one can obtain a release profile that depends more on the degradation of the polymer than the diffusion of the beneficial agent from the composition or vice versa. In this respect, at lower beneficial agent loading rates, one generally obtains a release profile reflecting degradation of the polymer wherein the release rate increases with time. At higher loading rates, one generally obtains a release profile caused by diffusion of the beneficial agent wherein the release rate decreases with time. At intermediate loading rates, one obtains combined release profiles so that if desired, a substantially constant release rate can be attained. While the particular release rate depends on the particular circumstances, such as the beneficial agent to be administered, release rates on the order of from about 0.1 micrograms/day to about 30 milligrams/day, preferably from about 1 microgram/day to about 20 milligrams per day, more preferably from about 10 micrograms/day to about 10 milligram/day, for periods of from about 24 hours to about 180 days, preferably 24 hours to about 120 days, more preferably 24 hours to about 90 days, often 3 days to about 90 days can be obtained. Further, the dose of beneficial agent may be adjusted by adjusting the amount of injectable depot gel injected.

To the extent that conventional optional ingredients are desired, such as hydroscopic agents, stabilizing agents and others, they are used in an amount that does not substantially affect the advantageous results which can be attained in accordance with the present invention. Other components may be present in the gel composition, to the extent they are desired or provide useful properties to the composition, such as polyethylene glycol, hydroscopic agents, stabilizing agents (for example surfactants like tween 20, tween 80, and the like, sugars such as sucrose, treholose, and the like, salts, antioxidants), pore forming agents, bulking agents (such as sorbitol, mannitol, glycine, and the like), chelating agents (such as divalent metal ions including zinc, magnesium, calcium, copper and the like), buffering agents (such as phosphate, acetane, succinate, histidine, TRIS, and the like) and others. When the composition includes a peptide or a protein that is soluble in or unstable in an aqueous environment, it may be highly desirable to include a solubility modulator that may, for example, be a stabilizing agent, in the composition. Various modulating agents are described in U.S. Patent Nos. 5,654,010 and 5,656,297, the disclosures of which are incorporated herein by reference. In the case of hGH, for example, it is preferable to include an amount of a salt of a divalent metal, preferably zinc. Examples of such modulators and stabilizing agents, which may form complexes with the beneficial agent or associate to provide the stabilizing or modulated release effect, include metal cations, preferably divalent, present in the composition as magnesium carbonate, zinc carbonate, calcium carbonate, magnesium acetate, magnesium sulfate, zinc acetate, zinc sulfate, zinc chloride, magnesium chloride, magnesium oxide, magnesium hydroxide, other antacids, and the like. The amounts of such agents used will depend on the nature of the complex formed, if any, or the nature of the association between the beneficial agent and the agent. Molar ratios of solubility modulator or stabilizing agent to beneficial agent of about 100:1 to 1:1, preferably 10:1 to 1:1, typically can be utilized.

Pore forming agents include biocompatible materials that when contacted with body fluids dissolve, disperse or degrade to create pores or channels in the polymer matrix. Typically, organic and non-organic materials that are water soluble such as sugars (e.g., sucrose, dextrose), water soluble salts (e.g., sodium chloride, sodium phosphate, potassium chloride, and sodium carbonate), water soluble solvents such as N-methyl-2-pyrrolidone and polyethylene glycol and water soluble polymers (e.g., carboxymethylcellulose, hydroxypropyl-cellulose, and the like) can conveniently be used as pore formers. Such materials may be present in amounts varying from about 0.1 % to about 100% of the weight of the polymer, but will typically be less than 50% and more typically less than 10-20% of the weight of polymer.

It is to be understood that the agent of the present invention does not constitute a mere diluent or a polymer-solvent that reduces viscosity by simply decreasing the concentration of the components of the composition. The use of conventional diluents can reduce viscosity, but can also cause the burst effect mentioned previously when the diluted composition is injected. In contrast, the injectable depot composition of the present invention can be formulated to avoid the burst effect by selecting the agent so that once injected into place, the agent has little impact on the release properties of the original system. Preferably, the system releases 40% or less by weight of the beneficial agent present in the viscous gel within the first 24 hours after implantation in the subject. More preferably, 30% or less by weight of the beneficial agent will be released within the first 24 hours after implantation, and the implanted composition has a burst index of 12 or less, preferably 8 or less.

To further understand the various aspects of the present invention, the results set forth in the previously described Figures were obtained in accordance with the following examples.

### Example 1

A gel vehicle for use in an injectable depot of the composition was prepared as follows. A glass vessel was tared on a Mettler PJ3000 top loader balance. Poly (D,L-lactide-co-glycolide) (PLGA), available as 50:50 Resomer^{®} RG502 (PLGA RG 502), 50:50 Resomer^{®} RG504 (PLGARG 504) or 50:50 DL-PLG with an inherent viscosity of 0.15 (PLGA-BPI, Birmingham Polymers, Inc., Birmingham, AL), was milled and sieved below 425 micron The polymer was weighed into the glass vessel. The glass vessel containing the polymer was tared and the corresponding solvent was added. Amounts expressed as percentages for various polymer/solvent combinations are set forth in Table 1, below. The polymer/solvent mixture was stirred at 250 ± 50 rpm (IKA electric stirrer, IKH-Werke GmbH & Co., Stanfen, Germany) for about 5 -10 minutes, resulting in a sticky paste-like substance containing polymer particles. The vessel containing the polymer/solvent mixture was sealed and placed in a temperature controlled incubator equilibrated to 37°C for 1 to 4 days, with intermittent stirring, depending on solvent and polymer type and solvent and polymer ratios. The polymer/solvent mixture was removed from the incubator when it appeared to be a clear amber homogeneous gel. Thereafter, the mixture was placed in an oven (65°C) for 30 minutes. It was noted that the PLGA-504 was dissolved in the mixture upon removal from the oven.

Additional depot gel vehicles are prepared with the following solvents or mixtures: benzyl benzoate ("BB"), ethanol, and propylene glycol ("PG"), and the following polymers: Poly (D,L-lactide) Resomer^{®} L104, PLA-L104, Poly (D,L-lactide-co-glycolide) 50:50 Resomer^{®} RG502, Poly (D,L-lactide-co-glycolide) 50:50 Resomer^{®} RG502H, PLGA-502H, Poly (D,L-lactide-co-glycolide) 50:50 Resomer^{®} RG503, PLGA-503, Poly L-Lactide MW 2,000 (Resomer^{®} L 206, Resomer^{®} L 207, Resomer^{®} L 209, Resomer^{®} L 214); Poly D,L Lactide (Resomer^{®} R 104, Resomer^{®} R 202, Resomer^{®} R 203, Resomer^{®} R 206, Resomer^{®} R 207, Resomer^{®} R 208); Poly L-Lactide-co-D,L-lactide 90:10 (Resomer^{®} LR 209); Poly D-L-lactide-co-glycolide 75:25 (Resomer^{®} RG 752, Resomer^{®} RG755, Resomer^{®} RG 756); Poly D,L-lactide-co-glycolide 85:15 (Resomer^{®} RG 858); Poly L-lactide-co-trimethylene carbonate 70:30 (Resomer^{®} LT 706); Poly dioxanone (Resomer^{®} X 210) (Boehringer Ingelheim Chemicals, Inc., Petersburg, VA); DL-lactide/glycolide 100:0 (MEDISORB^{®} Polymer 100 DL High, MEDISORB^{®} Polymer 100 DL Low); DL-lactide/ glycolide 85/15 (MEDISORB^{®} Polymer 8515 DL High, MEDISORB^{®} Polymer 8515 DL Low); DL-lactide/glycolide 75/25 (MEDISORB^{®} Polymer 7525 DL High, MEDISORB^{®} Polymer 7525 DL Low); DL-lactide/glycolide 65/35 (MEDISORB^{®} Polymer 6535 DL High, MEDISORB^{®} Polymer 6535 DL Low); DL-lactide/glycolide 54/46 (MEDISORB^{®} Polymer 5050 DL High, MEDISORB^{®} Polymer 5050 DL Low); and DL-lactide/glycolide 54/46 (MEDISORB^{®} Polymer 5050 DL 2A(3), MEDISORB^{®} Polymer 5050 DL 3A(3), MEDISORB^{®} Polymer 5050 DL 4A(3)) (Medisorb Technologies International L.P., Cincinatti, OH); and Poly D,L-lactide-co-glycolide 50:50; Poly D,L-lactide-co-glycolide 65:35; Poly D,L-lactide-co-glycolide 75:25; Poly D,L-lactide-co-glycolide 85:15; Poly DL-lactide; Poly L-lactide; Poly glycolide; Poly ε-caprolactone; Poly DL-lactide-co-caprolactone 25:75; and Poly DL-lactide-co-caprolactone 75:25 (Birmingham Polymers, Inc., Birmingham, AL). Typical polymer molecular weights were in the range of 14,400 - 39,700 (M_{w}) [6,400-12,200 (Mₙ)]. Representative gel vehicles are described in Table 1 below.

**Table 1**

| Formulation | Polymer¹ (%) | Benzyl Benzoate (%) | Ethanol (%) |
|---|---|---|---|
| 1 | 50 | 50 | 0 |
| 2 | 50 | 47.5 | 2.5 |
| 3 | 50 | 45 | 5 |
| 4 | 50 | 42.5 | 7.5 |

| | | | |
|---|---|---|---|
| 1 = PLGA- RG502 | | | |

### Example 2

### hGH Particle Preparation

Human growth hormone (hGH) particles (optionally containing zinc acetate) were prepared as follows:
hGH solution (5 mg/ml) solution in water (BresaGen
Corporation, Adelaide, Australia) was concentrated to 10 mg/mL using a Concentration/ Dialysis Selector diafiltering apparatus. The diafiltered hGH solution was washed with 5 times volume of tris or phosphate buffer solution (pH 7.6). Particles of hGH were then formed by spray drying or lyophilization using conventional techniques. Phosphate buffer solutions (5 or 50 mM) containing hGH (5 mg/mL) (and optionally various levels of zinc acetate (0 to 30 mM) when Zn complexed particles were prepared) were spray-dried using a Yamato Mini Spray dryer set at the following parameters:

| Spray Dryer Parameter | Setting |
|---|---|
| Atomizing Air | 2 psi |
| Inlet Temperature | 120°C |
| Aspirator Dial | 7.5 |
| Solution Pump | 2-4 |
| Main Air Valve | 40-45 psi |

Lyophilized particles were prepared from tris buffer solutions (5 or 50 mM: pH 7.6) containing hGH (5 mg/mL) using a Durastop µP Lyophilizer in accordance with the following freezing and drying cycles:

| | |
|---|---|
| Freezing cycle | Ramp down at 2.5 C/min to -30° C and hold for 30 min |
| | Ramp down at 2.5 C/min to -30° C and hold for 30 min |
| Drying cycle | Ramp up at 0.5 C/min to 10° C and hold for 960 min |
| | Ramp up at 0.5 C/min to 20° C and hold for 480 min |
| | Ramp up at 0.5 C/min to 25° C and hold for 300 min |
| | Ramp up at 0.5 C/min to 30° C and hold for 300 min |
| | Ramp up at 0.5 C/min to 5° C and hold for 5000 min |

### Example 3

### HGH-Stearic Acid Particle Preparation

Human growth hormone (hGH) particles were prepared as follows: Lyophilized hGH (3.22 grams, Pharmacia-Upjohn, Stockholm, Sweden) and stearic acid (3.22 grams, 95% pure, Sigma-Aldrich Corporation, St. Louis, MO) were blended and ground. The ground material was compressed in a 13 mm round die, with a force of 10,000 pounds for 5 minutes. Compressed tablets were ground and sieved through a 70 mesh screen followed by a 400 mesh screen to obtain particles having a size range between 38 - 212 microns.

### Example 4

### Drug Loading

Compressed particulates comprising beneficial agent/stearic acid prepared as above are added to a gel vehicle in an amount of 10 - 20 % by weight and blended manually until the dry powder is wetted completely. Then, the milky light yellow particle/gel mixture is thoroughly blended by conventional mixing using a Caframo mechanical stirrer with an attached square-tip metal spatula. Resulting formulations (6-12) are illustrated in Table 2 below. Final homogenous gel formulations were transferred to 3, 10 or 30 cc disposable syringes for storage or dispensing.

**Table 2**

| Formulation | Polymer¹ (%) | Benzyl Benzoate (%) | Ethanol (%) |
|---|---|---|---|
| 5² | 45.0 | 45.0 | 0.0 |
| 6³ | 40.0 | 40.0 | 0.0 |
| 7³ | 45.0 | 44.0 | 1.0 |
| 8³ | 39.0 | 39.0 | 2.7 |
| 9² | 39.0 | 39.7 | 0.0 |
| 10³ | 31.9 | 47.6 | 0.3 |
| 11³ | 33.5 | 44.0 | 0.3 |
| 12³ | 40.2 | 36.0 | 0.9 |
| 13³ | 32.4 | 44.2 | 1.2 |
| 14³ | 32.3 | 44.0 | 1.3 |
| 15³ | 36.2 | 39.6 | 1.5 |
| 16³ | 32.9 | 40.1 | 1.9 |
| 17⁴ | 35.3 | 45.8 | 0.9 |

| | | | |
|---|---|---|---|
| 1 = PLGA-502 polymer; 2 = 10 % particle loading (2.8% hGH, 5% stearic acid); 3 = 20 % particle loading (5% hGH, 10% stearic acid); 4 = 15 % particle loading (5% hGH, 7% stearic acid). | | | |

A representative number of implantable gels were prepared in accordance with the foregoing procedures and tested for in vitro release of beneficial agent as a function of time and also in in vivo studies in rats to determine release of the beneficial agent as determined by blood serum concentrations of beneficial agent as a function of time.

### Example 5

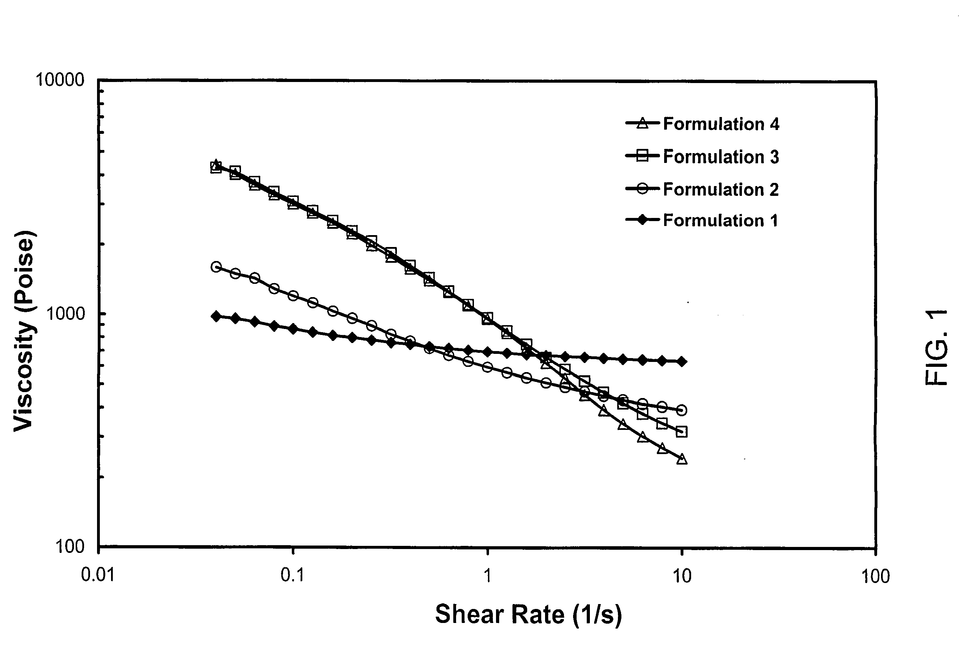

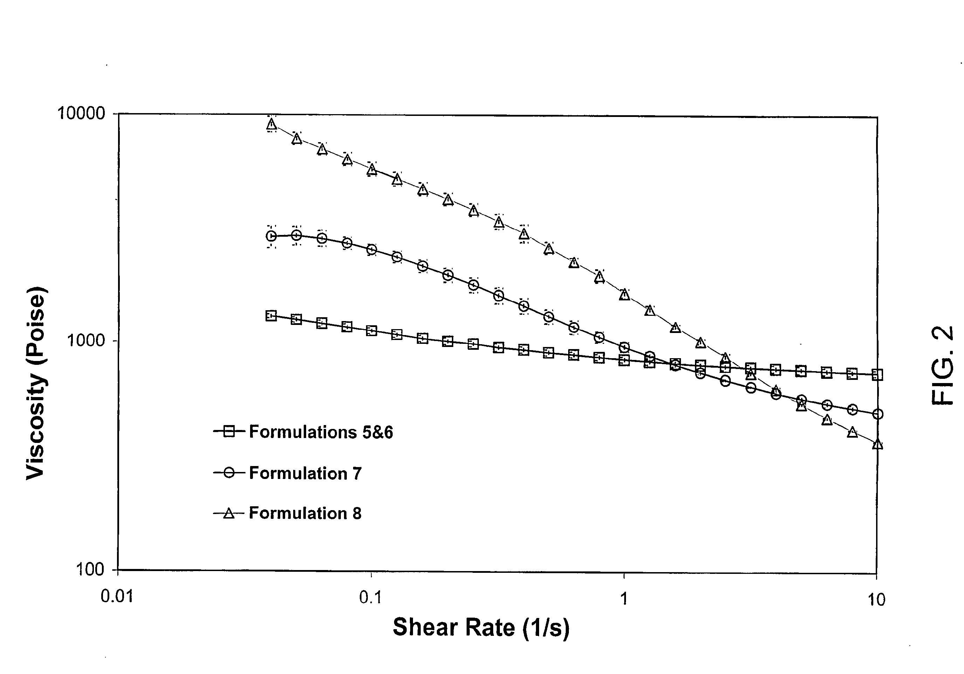

Rheological behavior was tested for depot vehicles and the depot formulations formulated with different solvents prepared as described above. Formulations 1-4 were tested for viscosity under various shear rates, and viscosity was measured using a Bohlin CVO50 rheometer (Bohlis Instruments Limited, Gloucestershire, UK) at 37°C. Figure 1 shows the viscosity of the gel formulations 1-4. Figure 2 shows the viscosity of the gel formulations 5-8. The viscosity at low shear rates represents the thickness of the gel formulation with minimal stress upon the formulation. As depicted in Figures 1 and 2, increasing the amount of ethanol in the formulation decreases the viscosity and increases shear thinning.

### Example 6

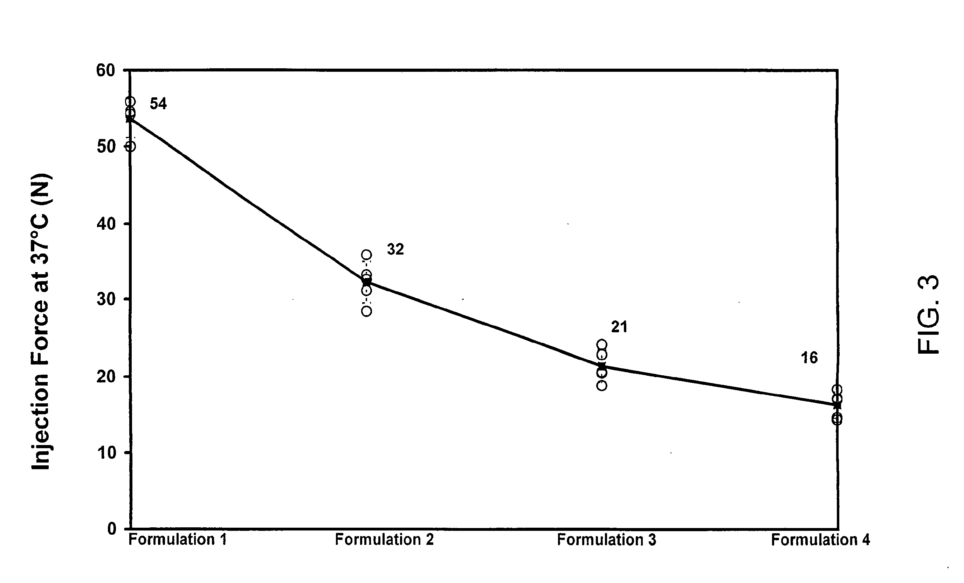

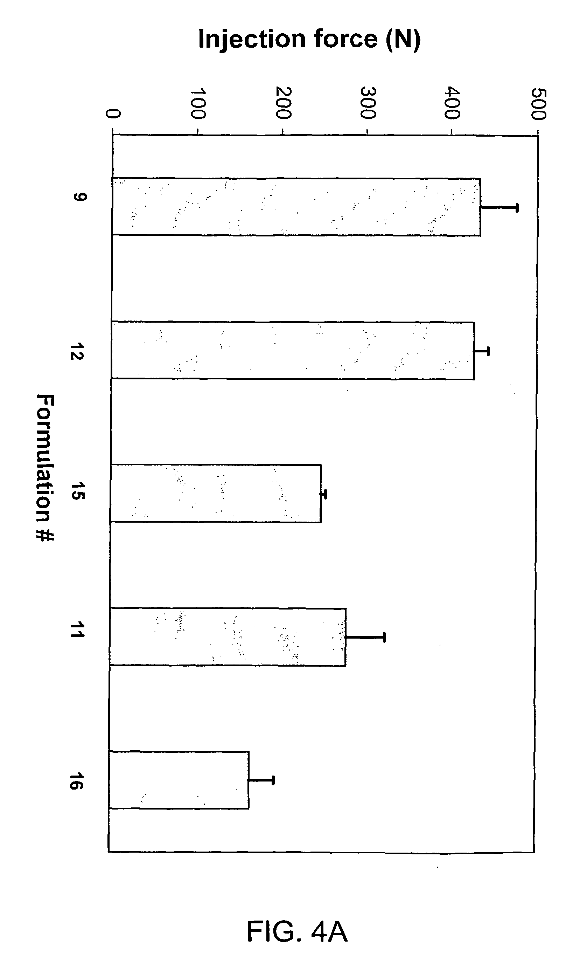

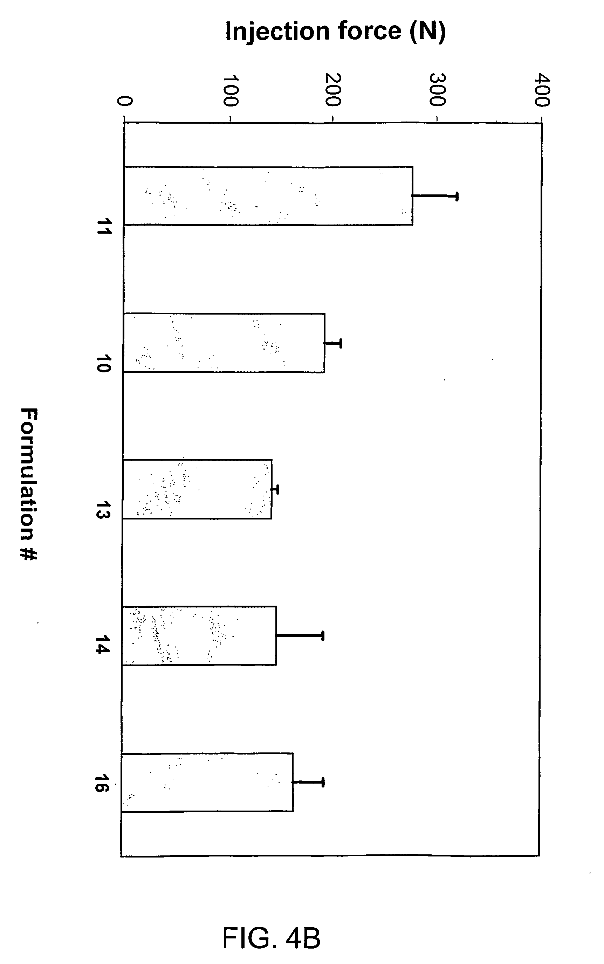

The injection force required to dispense depot vehicles and the depot formulations was evaluated for the formulations 1-4 and 5-12, respectively. The formulations were loaded in a Hamilton 500µl Gastight^{®} syringe (Hamilton, Reno, NV). Figure 3 is a graph illustrating the relationship between force required to inject Formulations 1-4 from a syringe using a 20 gauge needle. As shown in Figure 3, injection force of the depot vehicle formulation is significantly reduced as the amount of ethanol in the composition is increased from 0% to 15% of the combined weight of the solvent and the agent. Figures 4A & 4B illustrate the relationship between force required to inject Formulations 9-16 from a syringe using a 24 gauge needle at 1 ml/minute, at room temperature. Notably, due to the shear thing behavior, the formulations using ethanol as a thixotropic agent showed significantly reduced injection force while maintaining viscosities equal to or greater than the formulations using benzyl benzoate, at lower shear rate; thus maintaining the intactness of the depot after injection into the animals.

### Example 7

### hGH In Vivo Studies

In vivo studies in rats were performed following an open protocol to determine serum levels of hGH upon systemic administration of hGH via the implant systems of this invention. Depot gel hGH formulations were loaded into customized 0.5 cc disposable syringes. Disposable 16 gauge needles were attached to the syringes and were heated to 37°C using a circulator bath. Depot gel hGH formulations were injected into immunosuppressed rats and blood was drawn at specified time intervals. All serum samples were stored at 4°C prior to analysis. Samples were analyzed for intact hGH content using a radio immuno assay (RIA). At the end of study the rats are euthanized for gross clinical observation and the depot was retrieved for intactness observations.

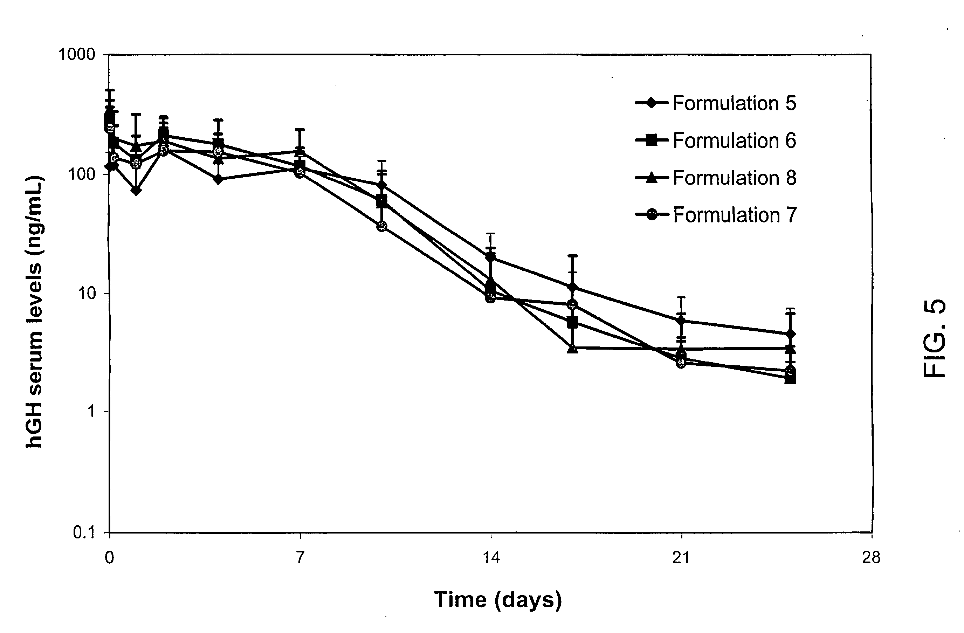

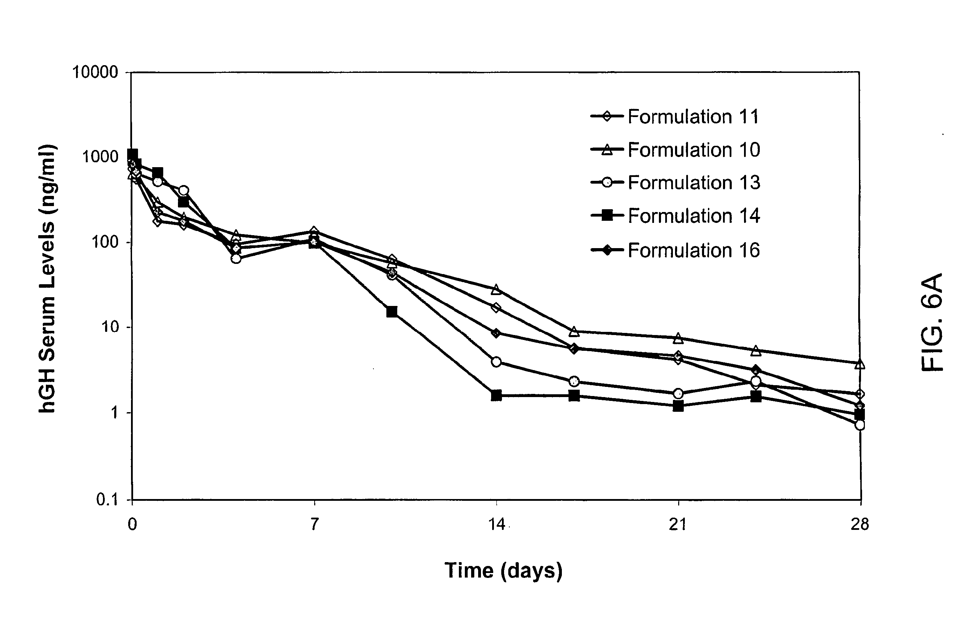

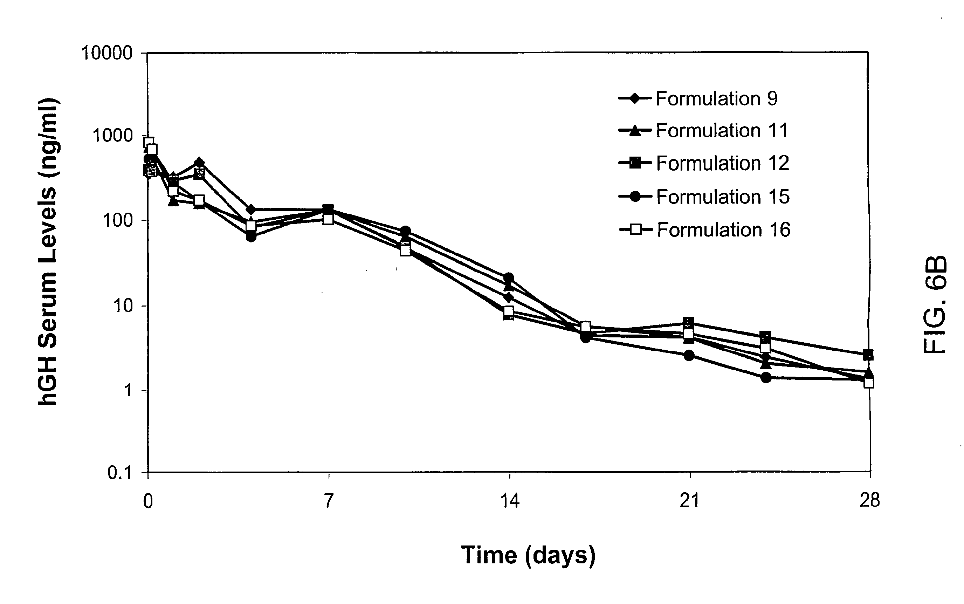

Figures 5, 6A and 6B illustrate representative in vivo release profile of human growth hormone ("hGH") obtained in rats from various depot formulation, including those of the present invention. The in vivo release profile of the depot formulations with ethanol are comparable to the control formulations (without ethanol). Thus, the depot formulations of the present invention reduce the injection force significantly without compromising the in vivo release profile of the beneficial agent.

At the end of study (i.e. at day 28) the depots were retrieved from the rats. Generally, a one-piece intact round-shaped depot was recovered corresponding to each injected depot in the animal.

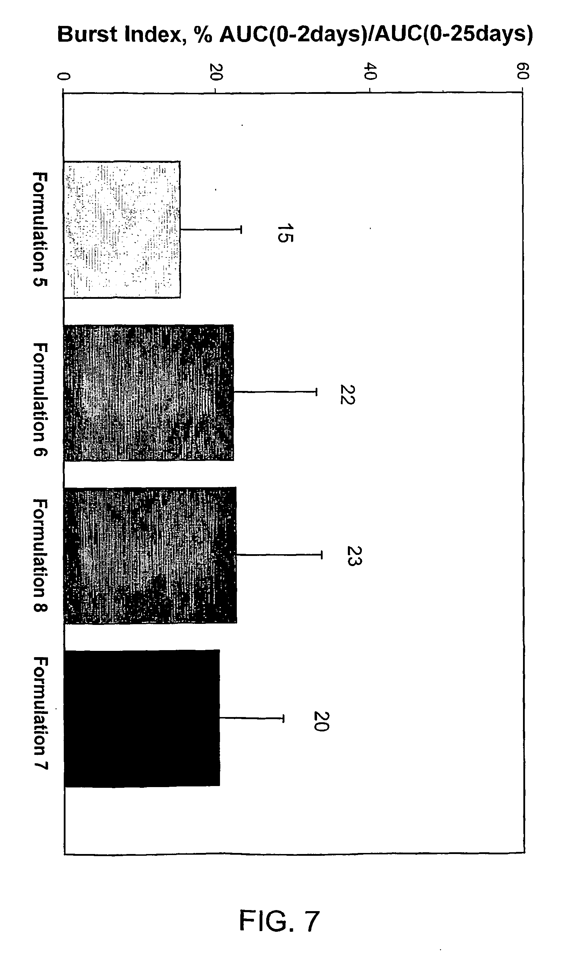

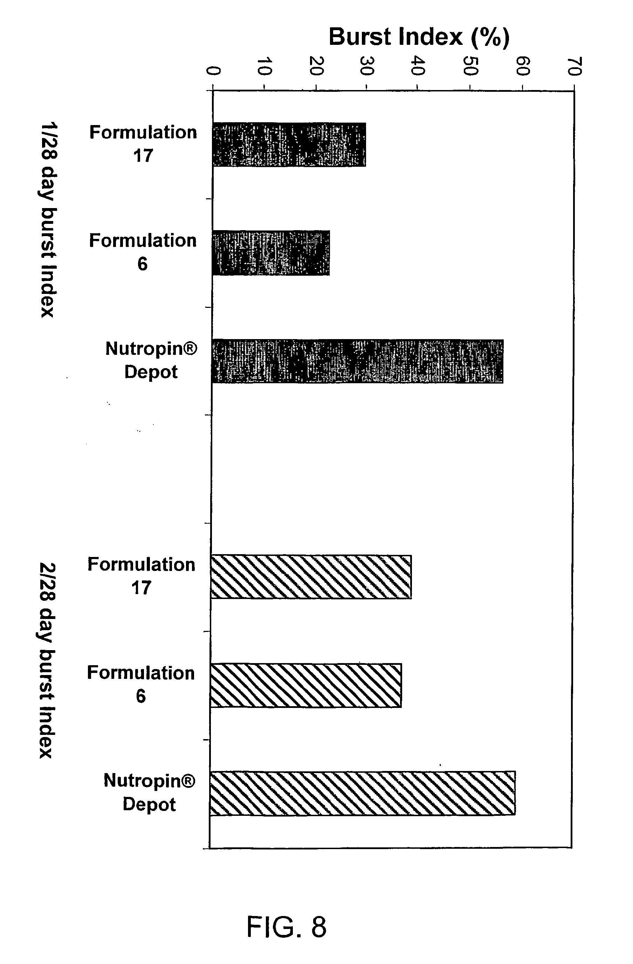

Figures 7 and 8 show the burst index profiling the release of human growth hormone ("hGH") obtained in rats from various depot formulation, including those of the present invention. The depot formulations of the present invention significantly reduce the injection force without compromising the in vivo release profile of the beneficial agent.

In accordance with various aspects of the present invention, one or more significant advantages can be obtained. More specifically, using simple processing steps, one can obtain a depot gel composition that can be injected into place in an animal without surgery using a low dispensing force through standard needles. Once in place, the composition will quickly return to its original viscosity and may exhibit rapid hardening so as to substantially avoid a burst effect and provide the desired beneficial agent release profile. Furthermore, once the beneficial agent has been fully administered, there is no need to remove the composition since it is fully biodegradable. As a still further advantage, the present invention avoids the use of microparticle or microcapsulation techniques which can degrade certain beneficial agents, like peptide and nucleic acid-based drugs and which microparticles and microcapsules maybe difficult to remove from the environment of use. Since the viscous gel is formed without the need for water, temperature extremes, or other solvents, suspended particles of beneficial agent remain dry and in their original configuration, which contributes to the stability of thereof. Further, since a mass is formed, the injectable depot gel composition may be retrieved from the environment of use if desired.

The invention comprises the following characteristics and features, either alone or in combination with one or more of each other:

a composition comprising a lactic acid-based polymer, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent; the composition wherein the agent is ethanol; the composition wherein the amount of ethanol is greater than or equal to 0.01 weight percent and less than or equal to 15 weight percent of the combined weight of the solvent and the agent; the composition wherein the amount of ethanol is greater than or equal to 0.1 weight percent and less than or equal to 5 weight percent of the combined weight of the solvent and the agent; the composition wherein the amount of ethanol that is greater than or equal to 0.5 weight percent and less than or equal to 5 weight percent of the combined weight of the solvent and the agent;

a composition comprising polylactic acid polymer, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent; the composition wherein the agent is ethanol; the composition wherein the amount of ethanol is greater than or equal to 0.01 weight percent and less than or equal to 15 weight percent of the combined weight of the solvent and the agent; the composition wherein the amount of ethanol is greater than or equal to 0.1 weight percent and less than or equal to 5 weight percent of the combined weight of the solvent and the agent; the composition wherein the amount of ethanol that is greater than or equal to 0.5 weight percent and less than or equal to 5 weight percent of the combined weight of the solvent and the agent; the composition wherein the weight average molecular weight of the polylactic acid polymer is between about 1,000 to about 120,000; preferably about 5,000 to about 50,000; and more preferably about 8,000 to about 30,000;

a composition comprising a lactic acid-based polymer formed as a copolymer of lactic acid and glycolic acid, a solvent that forms a polymer solution with said polymer, and an amount of an agent mixed with the polymer solution effective to form a thixotropic composition, said agent being selected from the group consisting essentially of lower alkanols and said amount being less than 15 weight percent of the combined weight of the solvent and the agent; the composition wherein the agent is ethanol; the composition wherein the amount of ethanol is greater than or equal to 0.01 weight percent and less than or equal to 15 weight percent of the combined weight of the solvent and the agent; the composition wherein the amount of ethanol is greater than or equal to 0.1 weight percent and less than or equal to 5 weight percent of the combined weight of the solvent and the agent; the composition wherein the amount of ethanol that is greater than or equal to 0.5 weight percent and less than or equal to 5 weight percent of the combined weight of the solvent and the agent; the composition wherein the weight average molecular weight of the copolymer is between about 1,000 to about 120,000; preferably about 5,000 to about 50,000; and more preferably about 8,000 to about 30,000.

The above-described exemplary embodiments are intended to be illustrative in all respects, rather than restrictive, of the present invention.

## Claims

1. A composition comprising a lactic acid-based polymer; a solvent that forms a polymer solution with said polymer; an amount of an agent mixed with the polymer solution effective to form a thixotropic gel composition, the solvent being selected from the group consisting of lower alkyl and aralkyl esters of benzoic acid, said agent being selected from the group consisting of alcohols that contain 2-6 carbon atoms, and said amount being less than 15 weight percent of the combined weight of the solvent and the agent; and an active substance.

2. The composition of claim 1 wherein the agent is ethanol.

3. The composition of claim 2 wherein the amount of ethanol is greater than or equal to 0.01 weight percent and less than or equal to 15 weight percent of the combined weight of the solvent and the agent.

4. The composition of claim 2 wherein the amount of ethanol is greater than or equal to 0.1 weight percent and less than or equal to 5 weight percent of the combined weight of the solvent and the agent.

5. The composition of claim 2 wherein the amount of ethanol is greater than or equal to 0.5 weight percent and less than or equal to 5 weight percent of the combined weight of the solvent and the agent.

6. The composition of claim 1, wherein the lactic acid-based polymer comprises a polylactic acid polymer.

7. The composition of claim 6 wherein the agent is ethanol.

8. The composition of claim 7 wherein the amount of ethanol is greater than or equal to 0.01 weight percent and less than or equal to 15 weight percent of the combined weight of the solvent and the agent.

9. The composition of claim 7 wherein the amount of ethanol is greater than or equal to 0.1 weight percent and less than or equal to 5 weight percent of the combined weight of the solvent and the agent.

10. The composition of claim 7 wherein the amount of ethanol is greater than or equal to 0.5 weight percent and less than or equal to 5 weight percent of the combined weight of the solvent and the agent.

11. The composition of claim 7 wherein the weight average molecular weight in the range of about 1,000 to about 120,000.

12. The composition of claim 1, wherein the lactic acid-based polymer is formed as a copolymer of lactic acid and glycolic acid.

13. The composition of claim 12 wherein the agent is ethanol.

14. The composition of claim 13 wherein the amount of ethanol is greater than or equal to 0.01 weight percent and less than or equal to 15 weight percent of the combined weight of the solvent and the agent.

15. The composition of claim 13 wherein the amount of ethanol is greater than or equal to 0.1 weight percent and less than or equal to 5 weight percent of the combined weight of the solvent and the agent.

16. The composition of claim 13 wherein the amount of ethanol is greater than or equal to 0.5 weight percent and less than or equal to 5 weight percent of the combined weight of the solvent and the agent.

17. The composition of claim 13 wherein the weight average molecular weight of the copolymer is between molecular weight in the range of about 1,000 to about 120,000.

18. The composition of claim 1 wherein the beneficial agent is selected from a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents, antiproliferative agents, antimitotic agents, angiogenic agents, anticoagulants, fibrinolytic agents, growth factors, antibodies, ocular drugs, and metabolites, analogs, derivatives, and fragments thereof.

19. The composition of claim 18 wherein the beneficial agent is present in an amount of from 0.1 to 50% by weight of the combined amounts of the polymer, the solvent and the beneficial agent.

20. The composition of claim 18 wherein the beneficial agent is in the form of particles dispersed or dissolved in the viscous gel.

21. The composition of claim 20 wherein the beneficial agent is in the form of particles wherein the particle further comprises a component selected from the group consisting of a stabilizing agent, bulking agent, cheating agent and a buffering agent.

22. The composition of claim 12 wherein the beneficial agent is selected from a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents, antiproliferative agents, antimitotic agents, angiogenic agents, anticoagulants, fibrinolytic agents, growth factors, antibodies, ocular drugs, and metabolites, analogs, derivatives, and fragments thereof.

23. The composition of claim 22 wherein the beneficial agent is present in an amount of from 0.1 to 50% by weight of the combined amounts of the polymer, the solvent and the beneficial agent.

24. The composition of claim 22 wherein the beneficial agent is in the form of particles dispersed or dissolved in the viscous gel.

25. The composition of claim 24 wherein the beneficial agent is in the form of particles wherein the particle further comprises a component selected from the group consisting of a stabilizing agent, bulking agent, chelating agent and a buffering agent.

## Patentansprüche

1. Eine Zusammensetzung umfassend:
ein auf Milchsäure basierendes Polymer,
ein Lösungsmittel, das eine Polymerlösung mit dem Polymer ausbildet,
eine Menge einer Agens, die mit der Polymerlösung gemischt wirksam eine thixotrophe Gelzusammensetzung ausbildet, während das Lösungsmittel ausgewählt ist aus der Gruppe bestehend aus niederen Alkyl und Aralkyesthern einer Benzoesäure, das Agens ist ausgewählt aus der Gruppe bestehend aus 2-6 Kohlenstoffatome enthaltenden Alkoholen und die Menge ist geringer als 15 Gewichtsprozent des kombinierten Gewichts von Lösungsmittel und Agens,
sowie eine aktive Substanz.

2. Die Zusammensetzung gemäß Anspruch 1, in der das Agens Ethanol ist.

3. Die Zusammensetzung gemäß Anspruch 2, in der die Menge an Ethanol größer oder gleich 0,01 Gewichtsprozent und kleiner oder gleich 15 Gewichtsprozent des kombinierten Gewichts von Lösungsmittel und Agens ist.

4. Die Zusammensetzung gemäß Anspruch 2, in der die Menge an Ethanol größer oder gleich 0,1 Gewichtsprozent und kleiner oder gleich 5 Gewichtsprozent des kombinierten Gewichts von Lösungsmittel und Agens ist.

5. Die Zusammensetzung gemäß Anspruch 2, in der die Menge an Ethanol größer oder gleich 0,5 Gewichtsprozent und kleiner oder gleich 5 Gewichtsprozent des kombinierten Gewichts von Lösungsmittel und Agens ist.

6. Die Zusammensetzung gemäß Anspruch 1, in der das auf Milchsäure basierende Polymer ein Polymilchsäurepolymer umfasst.

7. Die Zusammensetzung gemäß Anspruch 6, in der das Agens Ethanol ist.

8. Die Zusammensetzung gemäß Anspruch 7, in der die Menge an Ethanol größer oder gleich 0,01 Gewichtsprozent und kleiner oder gleich 15 Gewichtsprozent des kombinierten Gewichts von Lösungsmittel und Agens ist.

9. Die Zusammensetzung gemäß Anspruch 7, in der die Menge an Ethanol größer oder gleich 0,1 Gewichtsprozent und kleiner oder gleich 5 Gewichtsprozent des kombinierten Gewichts von Lösungsmittel und Agens ist.

10. Die Zusammensetzung gemäß Anspruch 7, in der die Menge an Ethanol größer oder gleich 0,5 Gewichtsprozent und kleiner oder gleich 5 Gewichtsprozent des kombinierten Gewichts von Lösungsmittels und Agens ist.

11. Die Zusammensetzung gemäß Anspruch 7, in der das durchschnittliche Molekulargewicht im Bereich von ungefähr 1.000 bis ungefähr 120.000 liegt.

12. Die Zusammensetzung gemäß Anspruch 1, in der das auf Milchsäure basierende Polymer als ein Co-Polymer von Milchsäure und Glykolsäure ausgebildet ist.

13. Die Zusammensetzung gemäß Anspruch 12, in der das Agens Ethanol ist.

14. Die Zusammensetzung gemäß Anspruch 13, in der die Menge an Ethanol größer oder gleich 0,01 Gewichtsprozent und kleiner oder gleich 15 Gewichtsprozent des kombinierten Gewichts von Lösungsmittel und Agens ist.

15. Die Zusammensetzung gemäß Anspruch 13, in der die Menge an Ethanol größer oder gleich 0,1 Gewichtsprozent und kleiner oder gleich 5 Gewichtsprozent des kombinierten Gewichts von Lösungsmittel und Agens ist.

16. Die Zusammensetzung gemäß Anspruch 13, in der die Menge an Ethanol größer oder gleich 0,5 Gewichtsprozent und kleiner oder gleich 5 Gewichtprozent des kombinierten Gewichts von Lösungsmittel und Agens ist.

17. Die Zusammensetzung gemäß Anspruch 13, in der das durchschnittliche Molekulargewicht des Co-Polymers zwischen dem Molekulargewicht im Bereich von ungefähr 1.000 bis 120.000 liegt.

18. Die Zusammensetzung gemäß Anspruch 1, in der das nutzbringende Agens ausgewählt ist aus einem Arzneimittel, Proteinen, Enzymen, Hormonen, Polynucleotiden, Nucleoproteinen, Polysacchariden, Glykoproteinen, Lipoproteinen, Polypeptiden, Steroiden, Analgetika, lokal Anästhetika, antibiotischen Agenzien, chemotherapeutischen Agenzien, immunosuppressiven Agenzien, entzündungshemmenden Agenzien, Antiwachstumsagenzien, antimitotischen Agenzien, angiogenischen Agenzien, Anticoagulanzien, fibrinolytischen Agenzien, Wachstumsfaktoren, Antikörpern, Augenarzneimitteln sowie Metaboliten, Analogon, Derivaten und Fragmenten davon.

19. Die Zusammensetzung gemäß Anspruch 18, in der das nutzbringende Agens in einer Menge von 0,1 bis 50 Gewichtsprozent der kombinierten Menge des Polymers, des Lösungsmittels und der nutzbringenden Agens vorliegt.

20. Die Zusammensetzung gemäß Anspruch 18, in der das nutzbringende Agens in der Form von dispergierten oder gelösten Partikeln in dem viskosen Gel vorliegt.

21. Die Zusammensetzung gemäß Anspruch 20, in der das nutzbringende Agens in der Form von Partikeln vorliegt, während die Partikel weiterhin eine Komponente umfassen, die ausgewählt ist aus der Gruppe bestehend aus einer Stabilisierungsagens, Quellungsagens, chelatbildender Agens und einer Pufferagens.

22. Die Zusammensetzung gemäß Anspruch 12, in der das nutzbringende Agens ausgewählt ist aus einem Arzneimittel, Proteinen, Enzymen, Hormonen, Polynucleotiden, Nucleoproteinen, Polysacchariden, Glykoproteinen, Lipoproteinen, Polypeptiden, Steroiden, Analgetika, lokale Anästhetika, antibiotischen Agenzien, chemotherapeutischen Agenzien, immunosuppressiven Agenzien, entzündungshemmenden Agenzien, Antiwachstumsagenzien, antimitotischen Agenzien, angiogenischen Agenzien, Anticoagulanzien, fibrinolytischen Agenzien, Wachstumsfaktoren, Antikörpern, Augenarzneimitteln sowie Metaboliten, Analogon, Derivaten und Fragmenten davon.

23. Die Zusammensetzung gemäß Anspruch 22, in der das nutzbringende Agens in einer Menge von 0,1 bis 50 Gewichtsprozent der kombinierten Menge des Polymers, des Lösungsmittels und der nutzbringenden Agens vorliegt.

24. Die Zusammensetzung gemäß Anspruch 22, in der das nutzbringende Agens in der Form von dispergierten oder gelösten Partikeln in dem viskosen Gel vorliegt.

25. Die Zusammensetzung gemäß Anspruch 24, in der das nutzbringende Agens in der Form von Partikeln vorliegt, während die Partikel weiterhin eine Komponente umfassen, die ausgewählt ist aus der Gruppe bestehend aus einer Stabilisierungsagens, Quellungsagens, chelatbildender Agens und einer Pufferagens.

## Revendications

1. Composition comprenant un polymère à base d'acide lactique ; un solvant qui forme une solution de polymère avec ledit polymère ; une quantité d'un agent mélangé avec la solution de polymère efficace pour former une composition de gel thixotrope, le solvant étant sélectionné dans le groupe constitué des esters d'aralkyle et d'alkyle inférieurs d'acide benzoïque, ledit agent étant sélectionné dans le groupe constitué d'alcools, qui contiennent 2 à 6 atomes de carbone, et ladite quantité étant inférieure à 15 pour cent en poids du poids combiné du solvant et de l'agent ; et une substance active.

2. Composition selon la revendication 1, dans laquelle l'agent est l'éthanol.

3. Composition selon la revendication 2, dans laquelle la quantité d'éthanol est supérieure ou égale à 0,01 pour cent en poids et inférieure ou égale à 15 pour cent en poids du poids combiné du solvant et de l'agent.

4. Composition selon la revendication 2, dans laquelle la quantité d'éthanol est supérieure ou égale à 0,1 pour cent en poids et inférieure ou égale à 5 pour cent en poids du poids combiné du solvant et de l'agent.

5. Composition selon la revendication 2, dans laquelle la quantité d'éthanol est supérieure ou égale à 0,5 pour cent en poids et inférieure ou égale à 5 pour cent en poids du poids combiné du solvant et de l'agent.

6. Composition selon la revendication 1, dans laquelle le polymère à base d'acide lactique comprend un polymère d'acide polylactique.

7. Composition selon la revendication 6, dans laquelle l'agent est l'éthanol.

8. Composition selon la revendication 7, dans laquelle la quantité d'éthanol est supérieure ou égale à 0,01 pour cent en poids et inférieure ou égale à 15 pour cent en poids du poids combiné du solvant et de l'agent.

9. Composition selon la revendication 7, dans laquelle la quantité d'éthanol est supérieure ou égale à 0,1 pour cent en poids et inférieure ou égale à 5 pour cent en poids du poids combiné du solvant et de l'agent.

10. Composition selon la revendication 7, dans laquelle la quantité d'éthanol est supérieure ou égale à 0,5 pour cent en poids et inférieure ou égale à 5 pour cent en poids du poids combiné du solvant et de l'agent.

11. Composition selon la revendication 7, dans laquelle la masse moléculaire moyenne en masse est dans la gamme d'environ 1 000 à environ 120 000.

12. Composition selon la revendication 1, dans laquelle le polymère à base d'acide lactique est sous la forme d'un copolymère d'acide lactique et d'acide glycolique.

13. Composition selon la revendication 12, dans laquelle l'agent est l'éthanol.

14. Composition selon la revendication 13, dans laquelle la quantité d'éthanol est supérieure ou égale à 0,01 pour cent en poids et inférieure ou égale à 15 pour cent en poids du poids combiné du solvant et de l'agent.

15. Composition selon la revendication 13, dans laquelle la quantité d'éthanol est supérieure ou égale à 0,1 pour cent en poids et inférieure ou égale à 5 pour cent en poids du poids combiné du solvant et de l'agent.

16. Composition selon la revendication 13, dans laquelle la quantité d'éthanol est supérieure ou égale à 0,5 pour cent en poids et inférieure ou égale à 5 pour cent en poids du poids combiné du solvant et de l'agent.

17. Composition selon la revendication 13, dans laquelle la masse moléculaire moyenne en masse du copolymère est dans la gamme d'environ 1 000 à environ 120 000.

18. Composition selon la revendication 1, dans laquelle l'agent bénéfique est sélectionné parmi un médicament, des protéines, des enzymes, des hormones, des polynucléotides, des nucléoprotéines, des polysaccharides, des glycoprotéines, des lipoprotéines, des polypeptides, des stéroïdes, des analgésiques, des anesthésiques locaux, des agents antibiotiques, des agents chimiothérapeutiques, des agents immunosuppresseurs, des agents anti-inflammatoires, des agents antiprolifératifs, des agents antimitotiques, des agents angiogéniques, des anticoagulants, des agents fibrinolytiques, des facteurs de croissance, des anticorps, des médicaments oculaires, et leurs métabolites, analogues, dérivés et fragments.

19. Composition selon la revendication 18, dans laquelle l'agent bénéfique est présent en une quantité d'environ 0,1 à 50 % en poids des quantités combinées du polymère, du solvant et de l'agent bénéfique.

20. Composition selon la revendication 18, dans laquelle l'agent bénéfique est sous la forme de particules dispersées ou dissoutes dans le gel visqueux.

21. Composition selon la revendication 20, dans laquelle l'agent bénéfique est sous la forme de particules, la particule comprenant en outre un composant sélectionné dans le groupe constitué d'un agent stabilisant, d'un diluant, d'un chélateur et d'un tampon.

22. Composition selon la revendication 12, dans laquelle l'agent bénéfique est sélectionné parmi un médicament, des protéines, des enzymes, des hormones, des polynucléotides, des nucléoprotéines, des polysaccharides, des glycoprotéines, des lipoprotéines, des polypeptides, des stéroïdes, des analgésiques, des anesthésiques locaux, des agents antibiotiques, des agents chimiothérapeutiques, des agents immunosuppresseurs, des agents anti-inflammatoires, des agents antiprolifératifs, des agents antimitotiques, des agents angiogéniques, des anticoagulants, des agents fibrinolytiques, des facteurs de croissance, des anticorps, des médicaments oculaires, et leurs métabolites, analogues, dérivés et fragments.

23. Composition selon la revendication 22, dans laquelle l'agent bénéfique est présent en une quantité d'environ 0,1 à 50 % en poids des quantités combinées du polymère, du solvant et de l'agent bénéfique.

24. Composition selon la revendication 22, dans laquelle l'agent bénéfique est sous la forme de particules dispersées ou dissoutes dans le gel visqueux.

25. Composition selon la revendication 24, dans laquelle l'agent bénéfique est sous la forme de particules, la particule comprenant en outre un composant sélectionné dans le groupe constitué d'un agent stabilisant, d'un diluant, d'un chélateur et d'un tampon.
